(19) Europäisches Patentamt

European Patent Office

Office européen des brevets

(11) **EP 1 854 469 A1**

(12) **EUROPEAN PATENT APPLICATION**
published in accordance with Art. 158(3) EPC

(43) Date of publication:
**14.11.2007 Bulletin 2007/46**

(51) Int Cl.:
*A61K 36/03* (2006.01)   *A61K 36/06* (2006.01)
*A61K 35/74* (2006.01)   *A61K 36/48* (2006.01)
*A61P 11/06* (2006.01)   *A61P 17/00* (2006.01)
*A61P 31/00* (2006.01)   *A61P 31/18* (2006.01)
*A61P 35/00* (2006.01)   *A61P 37/02* (2006.01)
*A61P 37/04* (2006.01)   *A61P 37/08* (2006.01)
*A23L 1/30* (2006.01)   *A23L 2/52* (2006.01)

(21) Application number: **06715170.4**

(22) Date of filing: **03.03.2006**

(86) International application number:
**PCT/JP2006/304087**

(87) International publication number:
**WO 2006/093267 (08.09.2006 Gazette 2006/36)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.03.2005 JP 2005061038**
**28.03.2005 JP 2005091729**

(71) Applicant: **SUNTORY LIMITED**
**Osaka-shi,**
**Osaka 530-8203 (JP)**

(72) Inventors:
• **NONAKA, Yuji**
**c/o Suntory Limited,**
**Shimamoto-cho, Mishima-gun, Osaka 618-8503 (JP)**

• **IZUMI, Fumi**
**c/o Suntory Limited,**
**Shimamoto-cho, Mishima-gun, Osaka 618-8503 (JP)**

• **IZUMO, Takayuki**
**c/o Suntory Limited,**
**Shimamoto-cho, Mishima-gun, Osaka 618-8503 (JP)**

(74) Representative: **Vossius & Partner**
**Siebertstrasse 4**
**81675 München (DE)**

(54) **FERMENTATION COMPOSITION HAVING IMMUNOMODULATING EFFECT**

(57)    Novel compositions are provided that exhibit immunomodulatory actions such as an immunostimulating action and an anti-allergic action. Compositions are provided in which Kombu fermented with lactic acid bacteria is contained either alone or in combination with sesame and soybean(s) that have been fermented with lactic acid bacteria. The compositions enable nutrients to be absorbed in a balanced way, feature high safety, and have superior immunomodulatory actions.

EP 1 854 469 A1

**Description**

TECHNICAL FIELD

The present invention relates to compositions having immunomodulatory actions that contain Kombu fermented with lactic acid bacteria; more specifically, it relates to compositions having an immunostimulating action and/or compositions having an anti-allergic action. The present invention also relates to compositions having immunomodulatory actions that contain Kombu fermented with lactic acid bacteria, sesame fermented with lactic acid bacteria, and soybean (s) fermented with lactic acid bacteria.

BACKGROUND ART

In recent years, problems associated with allergy are becoming increasingly evident at an accelerated rate. Allergy causes various diseases and topped with pollinosis, diseases such as atopic dermatitis, food allergy, allergic rhinitis, and allergic asthma are known to afflict particularly large numbers of patients; notably, it has been reported that the number of patients with pollinosis has increased drastically. Faced with this current status, many companies, most of which are pharmaceutical manufacturers, have been conducting intensive studies for combating allergy, and a number of products have been developed. Representative of them are antiallergics and antihistamines; these drugs are characterized by presenting an action on the release of inflammatory substances from granulocytes, called mast cells and located at the terminal end of the mechanism for the manifestation of allergy, as well as an action on the associated receptors; they bring about temporary palliation of symptoms but, on the other hand, they have no effect on the basics of the manifestation of allergy. Patients showing particularly serious symptoms are occasionally administered with steroids, but this type of drugs have posed the problem of side-effects. Under these circumstances, there is a strong need for a radical method of treating allergy and a method of preventing it. With an aging society being ushered in, Japan is now seeing a growing increase in the number of deaths among elderly persons, from infections that are attributable to their immune system that weakens as the result of aging. Immunity is also known to decrease in our hectic society and stressful environment; and taking some measure against this situation is strongly desired.

In order to cope with these problems, it is held urgent to develop a safe composition for oral intake, which is manufactured with common foodstuffs, exhibits mild immunomodulatory actions for a prolonged time, and is capable of maintaining people's health in a stressful environment, as a means of enhancing their immunomodulatory function, whereby their immunity is sufficiently improved that it can prevent an infection, or prevent or treat allergy.

Japan has a long established custom of eating seaweeds, and Kombu in particular is held as a good-luck food or a macrobiotic food, and is also a traditional foodstuff that can be included in a vegetarian dish. Seaweeds as food are rich not only in minerals that are prone to be deficient, such as calcium, sodium, magnesium, phosphorus, iron and iodine, but also in a variety of vitamins and dietary fibers such as fucoidin; they have also been shown to have immunomodulatory actions. As for lactic acid fermented foods, the recognition that eating them is good for health is growing increasingly in parallel with the accumulation of supportive scientific observations; and the immunomodulatory actions of various lactic acid bacteria and/or lactic acid fermented foods have also become clear. However, neither seaweeds nor lactic acid fermented foods can generally be said to have adequate immunomodulatory action when taken alone. Therefore, a lactic acid fermented foodstuff of seaweed origin that has those two features in combination is considered to be an attractive foodstuff that is expected to improve health functions. However, Kombu and other seaweeds are short of the nutrients that are required for implantation and growth of fermentation microbial strains. What is more, a lot of time, labor and cost is required to cultivate seaweeds. Therefore, fermented foods of seaweed origin and their physiological activities have not been fully studied.

As regards seaweed ferments, Patent Document 1 discloses a fermented food of seaweed origin that is obtained by treating a seaweed with cellulase or the like to convert it to single cells, for its saccharification, namely, for the supply of sugars serving as a fermentation substrate, and then fermenting it with lactic acid bacteria and/or a yeast. This document shows that *Laminaria japonica* and *Undaria pinnatifida* were fermented by the disclosed method, and it also states that a fermented food of seaweed origin, specifically, a food fermented by treating *Undaria pinnatifida* with cellulase and inoculating it with three kinds of microorganisms (one kind of lactic acid bacteria, *Lactobacillus brevis* strain NRIFS B5201, and two kinds of yeasts, *Debaryomyces hansenii* strain NRIFSY5201, and *Candida zeylanoides* strain NRIF SY5206) has a blood triglyceride lowering action and a liver lipid lowering action.

In addition, Patent Document 2 discloses a fermented food that is obtained by first allowing a mixture of soybean (s) and Kombu to be ingested with a microorganism of the genus *Rhyzopus,* fermenting the mixture, and mixing the ferment with unfermented sesame; and discloses that the food can be easily ingested to show high nutritional values and absorbability while exhibiting good taste.

Patent Document 1: JP 2003-201 A

Patent Document 2: Japanese Patent 2846547B

DISCLOSURE OF THE INVENTION

PROBLEMS TO BE SOLVED BY THE INVENTION

An object of the present invention is to provide compositions that are highly safe and have superior immunomodulatory actions by using common foodstuffs, and imparting an immunomodulatory function to them or enhancing their immunomodulatory function.

MEANS FOR SOLVING THE PROBLEMES

The present inventors used lactic acid bacteria of plant origin to ferment Kombu, which is commonly held to defy fermentation, and successfully obtained its lactic acid bacterial fermentation product. The present inventors then conducted extensive studies on what physiological activities that lactic acid bacterial fermentation product of Kombu would have when it was ingested orally; as a result, they found that said fermentation product showed markedly improved immunomodulatory actions (immunostimulating action and immuno-balance adjusting action) as compared with the case when Kombu alone and/or the lactic acid bacteria alone was ingested. In addition, the present inventors made intensive studies toward the development of foodstuffs that could allow this lactic acid bacterial fermentation product of Kombu to exhibit its immunomodulatory function more efficiently and which also had high nutritional values; as it turned out, a composition comprising Kombu fermented with lactic acid bacteria, in combination with sesame fermented with lactic acid bacteria, and soybean(s) fermented with lactic acid bacteria surprisingly showed unpredictably high immunomodulatory actions such as immunostimulating and ant-allergic actions when it was ingested orally. These immunomodulatory actions in turn were found to be considerably greater than the actions of lactic acid bacteria alone or the unfermented starting materials for fermentation alone. What is more, these immunomodulatory actions were maintained even after the composition of the present invention was sterilized with heat.
The present invention has been accomplished on the basis of those surprising findings.
The present invention provides:

1. A composition having an immunomodulatory action that comprises Kombu fermented with lactic acid bacteria;
2. A composition as recited in 1, wherein the immunomodulatory action is an immunostimulating action and/or an anti-allergic action;
3. A composition as recited in 1 or 2, wherein the lactic acid bacteria are those of plant origin;
4. A composition as recited in any one of 1 to 3, wherein a single dosage thereof contains at least 5 mg of the Kombu fermented with lactic acid bacteria;
5. A composition comprising Kombu fermented with lactic acid bacteria, sesame fermented with lactic acid bacteria, and soybean(s) fermented with lactic acid bacteria;
6. A composition as recited in 5, wherein the lactic acid bacteria are those of plant origin;
7. A composition as recited in 5 or 6, which has an immunomodulatory action;
8. A composition as recited in 7, wherein the immunomodulatory action is an immunostimulating action and/or an anti-allergic action;
9. A composition as recited in any one of 5 to 9, wherein a single dosage thereof contains the Kombu fermented with lactic acid bacteria, the sesame fermented with lactic acid bacteria and the soybean(s) fermented with lactic acid bacteria in a total amount of 1 to 10000 mg, preferably 10 to 10000 mg;
10. A composition as recited in any one of 1 to 9, which is a food or beverage, a food additive, a health food, or a pharmaceutical composition;
11. A composition as recited in any one of 1 to 10, which is in the form of a pill;
12. A food or beverage comprising Kombu fermented with lactic acid bacteria as an active ingredient, and having an indication stating that it has an immunomodulatory action.

EFFECT OF THE INVENTION

The compositions of the present invention are manufactured with foodstuffs, so they feature high safety and can be ingested continuously for a short or long period either on a daily basis or at suitable intervals of days. Therefore, if they are ingested as foods or beverages or as health foods, the compositions modulate immune function over a prolonged period, whereby the occurrence of compromised immune function due to various factors can be prevented. In addition, the compositions can modulate the balance in immune function, to prevent an excessive increase in immune function that will adversely affect the living body.

In addition, when administering the compositions of the present invention as pharmaceuticals, various symptoms due to compromised immune function or an excessive increase in immune function can be mitigated or cured with mild effect.

As a further advantage, the compositions of the present invention also enable efficient utilization of the superior nutritional values and functionality of Kombu, soybean(s), and sesame.

BRIEF DESCRIPTION OF THE DRAWINGS

[FIG. 1] FIG. 1 is a graph showing the results obtained by measuring the amounts of IL-12 production from mouse-derived macrophages that were treated with strain S-PT84, unfermented Kombu, fermented Kombu, or fermented sesame/soybean/Kombu.

[FIG. 2] FIG. 2 is a graph showing the amounts of IL-12 production by macrophages derived from mice that were allowed to ingest unfermented Kombu, fermented Kombu, unfermented sesame/soybean/Kombu, or fermented sesame/soybean/Kombu.

[FIG. 3] FIG. 3 is a graph showing the NK activity of spleen lymphocytes derived from mice that were allowed to ingest S-PT84, unfermented Kombu, or fermented Kombu.

[FIG. 4] FIG. 4 is a graph showing the amounts of IFN-γ or IL-4 production by spleen lymphocytes derived from mice that were allowed to ingest S-PT84, unfermented Kombu, or fermented Kombu.

[FIG. 5] FIG. 5 is a graph showing the Th1/Th2 balance of spleen lymphocytes derived from mice that were allowed to ingest S-PT84, unfermented Kombu, or fermented Kombu.

[FIG. 6] FIG. 6 is a graph showing the results obtained by measuring the NK activity of spleen cells derived from mice that were allowed to ingest S-PT84 or the fermented composition orally.

[FIG. 7] FIG. 7 is a graph showing the results obtained by measuring the IL-4 production from spleen cells derived from mice that were allowed to ingest S-PT84 or the fermented composition orally.

[FIG. 8] FIG. 8 is a graph showing the results obtained by measuring the IFN-γ production from spleen cells derived from mice that were allowed to ingest S-PT84 or the fermented composition orally.

[FIG. 9] FIG. 9 is a graph showing the results obtained by measuring the NK activity of spleen cells derived from mice that were allowed to ingest S-PT84 or the fermented composition orally under stress.

[FIG. 10] FIG. 10 is a graph showing the results obtained by measuring the IL-4 production from spleen cells derived from mice that were allowed to ingest S-PT84 or the fermented composition orally under stress.

[FIG. 11] FIG. 11 is a graph showing the results obtained by measuring the IFN-γ production from spleen cells derived from mice that were allowed to ingest S-PT84 or the fermented composition orally under stress.

[FIG. 12] FIG. 12 is a graph showing the results obtained by measuring the total IgE concentration in OVA-induced allergic mice that were allowed to orally ingest S-PT84, the unfermented composition, S-PT84 + the unfermented composition, or the fermented composition.

[FIG. 13] FIG. 13 is a graph showing the results obtained by measuring the IL-4 production from spleen cells derived from OVA-induced allergic mice that were allowed to orally ingest S-PT84 + the unfermented composition or the fermented composition.

[FIG. 14] FIG. 14 is a graph showing the results obtained by measuring the IFN-γ production from spleen cells derived from OVA-induced allergic mice that were allowed to orally ingest S-PT84 + the unfermented composition or the fermented composition.

[FIG. 15] FIG. 15 shows the results obtained by measuring the total IgA level in the intestinal lumen of mice that were allowed to orally ingest S-PT84 or the fermented composition.

[FIG. 16] FIG. 16 is a graph showing the results obtained by measuring the IL-4 production from Peyer's patch cells derived from mice that were allowed to orally ingest S-PT84 or the fermented composition.

[FIG. 17] FIG. 17 is a graph showing the results obtained by measuring the IFN-γ production from Peyer's patch cells derived from mice that were allowed to ingest S-PT84 or the fermented composition orally.

[FIG. 18] FIG. 18 shows the results of measuring the size of Sarcoma 180 tumor in mice that were allowed to orally ingest unfermented Kombu or fermented Kombu.

[FIG. 19] FIG. 19 is a graph showing the results of measuring the NK activity in humans who were allowed to orally ingest fermented pills.

[FIG. 20] FIG. 20 is a graph showing changes in the NK activity in humans who were allowed to ingest the fermented pills.

[FIG. 21] FIG. 21 is a graph showing changes in the Th1/Th2 ratio in humans who were allowed to ingest the fermented pills.

## BEST MODE FOR CARRYING OUT THE INVENTION

Kombu fermented with lactic acid bacteria

Kombu as referred to in the present invention is a generic name for the class *Phaeophyceae,* family Laminariaceae, genus *Laminaria spp.,* and their related species, and they include, but are not limited to, *L. japonica, L. japonica var. ochotensis, L. angustata, L. angustata var. longissima, L. religiosa, Arthrothamnus bifidus, Kjellmaniella gyrata,* and *Kjellmaniella crassifolia.*

Kombu as the starting material for the Kombu fermented with lactic acid bacteria according to the present invention (lactic acid bacteria fermented Kombu) may be any of the Kombu species mentioned above, either fresh or dried, which is used after removing any foreign matter such as sand. Because of ease in fermentation, shredded or pulverized Kombu is preferably used and, if desired, it may be used after being flavored with salt, sugar, amino acid, organic acid, nucleic acid, etc. Such Kombu is commonly available on the market and exemplary trade names include "KOMBU POWDER" (Yaizu Suisankagaku Industry Co., Ltd.), "KOMBU POWDER" (Souman Suisan, Co., Ltd.), etc.

The lactic acid bacteria used to ferment the starting material Kombu are not limited in any particular way as long as they can ferment Kombu, and those which are prepared by freeze drying in the usual manner or those which are stored chilled may be employed. The lactic acid bacteria include, for example, the genera *Lactobacillus, Lactococcus, Streptococcus, Bifidobacterium,* and *Leuconostoc;* from among these, one or more lactic acid bacteria may be chosen and used either simultaneously or consecutively. Among others, isolated lactic acid bacteria that feed on plant components to live (lactic acid bacteria of plant origin) are used advantageously for various reasons such as: their ability to ferment even starting materials of plant origin such as Kombu that have no abundant supply of nutrients; their easy availability as food due to their resistibility to acid and alkali, and their capability of surviving in the presence of sodium chloride at high concentration; and their tolerance in the intestinal system which is claimed to have utility in humans. Such lactic acid bacteria of plant origin may be exemplified by *Lactobacillus plantarum, Lactobacillus pentosus, Lactobacillus brevis, Lactobacillus fermentum, Tetragenococcus halophilus, Pediococcus pentosaceus,* etc. It should be noted here that the Assignee of the subject application previously isolated from Kyoto pickles (Shibazuke) various lactic acid bacteria of plant origin having immunomodulatory actions (see JP 2005-333919 A). In the present invention, those lactic acid bacteria can also be used advantageously.

In addition, lactic acid bacteria cause either homo-lactic acid fermentation or hetero-lactic acid fermentation, and the lactic acid bacteria to be used in the present invention may be of either fermentation type; acetic acid, carbon dioxide gas, alcohol, etc. that are generated during hetero-lactic acid fermentation can also be used for the purpose of imparting taste or aroma, but if carbon dioxide gas is generated in a large volume, it might deteriorate the handling in the process of manufacture using a fermentation tank and the like. On the other hand, homo-lactic acid fermentation can be used advantageously in the present invention, since the fermentation generates a sufficiently large volume of lactic acid to reduce the risk of putrefaction which may be caused in the process of fermentation due to the action of putrefying bacteria and the like, derived from Kombu.

To produce the lactic acid bacteria fermented Kombu of the present invention, water is usually added to the starting material Kombu, which is inoculated with the above-mentioned lactic acid bacteria and fermented, with the ferment being subsequently heated or irradiated (preferably heated) for sterilization. The sterilized ferment is filtered or centrifuged (preferably filtered) and almost all dead microbes are removed, whereby the lactic acid bacteria fermented Kombu can be obtained.

Before fermentation, a sterilizing treatment is preferably performed. The temperature and time for the sterilizing treatment may be set as appropriate, and it may be exemplified by one of holding at 90°C for 10 minutes. In addition, during the fermentation of Kombu, polysaccharides derived from the Kombu may sometimes render the fermentation liquor considerably viscous; if this occurs, an additive such as an enzyme like alginate lyase may be added to improve the operating efficiency.

The fermentation treatment is performed by adding a starter including lactic acid bacteria to the preliminarily sterilized Kombu mentioned above. In this case, a saccharide such as glucose or a nitrogen source such as soybean peptide is preferably added as a fermentation promoting additive. The temperature and time for the fermentation treatment may be set as appropriate depending on the nature of the lactic acid bacteria to be used. The degree of fermentation can be evaluated by measuring the pH of the Kombu being subjected to fermentation treatment and in the case of the lactic acid bacteria fermented Kombu of the present invention, fermentation is preferably continued until its pH becomes 5.0 or below.

The thus obtained lactic acid bacteria fermented Kombu may be used as such in a liquid form, but from the viewpoint of ease in operation and storage, it is preferably spray dried or freeze dried into a powder form.

Lactic acid bacteria fermented sesame/soybean/Kombu (Kombu, sesame and soybean(s) that have been fermented with lactic acid bacteria)

The present invention is characterized in that the lactic acid bacteria fermented Kombu described above is further combined with sesame fermented with lactic acid bacteria and soybean(s) fermented with lactic acid bacteria, to potentiate the immunomodulatory actions.

Here, sesame as referred to in the present invention means seeds of plants, *Sesamun indicum,* and they include, but are not limited to, white sesame, black sesame, yellow sesame, brown sesame, etc. These sesames may be used either alone in admixture of two or more kinds. The form of the sesame that serves as the starting material for the sesame fermented with lactic acid bacteria according to the present invention (the lactic acid bacteria fermented sesame) is not limited, and any form such as grains, a ground product, paste, etc. can be employed; however, because of ease in fermentation, a ground product or paste of sesame is preferably used. A lot of sesame pastes are commercially available under various trade names including, for example, "JUN-NERI GOMA-KURO" (TAKEMOTO OIL & FAT Co., Ltd.). In order to obtain a lactic acid bacteria fermented sesame with low fat content, defatted sesame may be used as the starting material, but from the viewpoints of the high nutritional values and functionality possessed by sesame, it is preferred to use a yet-to-be defatted sesame having oil content.

Soybean(s) as referred to in the present invention means seeds of plants, *Glycine max,* and they include a large number of varieties that differ with the growth period and place of production. In the present invention, any variety can be used without any particular limitation. Soybeans that serve as the starting material for the soybean(s) fermented with lactic acid bacteria according to the present invention (the lactic acid bacteria fermented soybean) include solid soybean(s), soybean flour, soybean oil cake, defatted soybean(s), toasted soybean flour, their hydrolyzates, etc. and these can be used without any particular limitation; for ease in fermentation, it is preferred to use soybeans in powdered form. Such soybean powders are commercially available under various trade names including "KURODAIZU KINAKO" (K. Kobayashi Co., Ltd.) and "PROLIFE" (RIKEN Nosan-Kako Co., Ltd.).. In order to obtain lactic acid bacteria fermented soybean(s) with low fat content, defatted soybean(s) may be used.

The present invention relates to a composition comprising the lactic acid bacteria fermented Kombu, the lactic acid bacteria fermented sesame, and the lactic acid bacteria fermented soybean(s) (the composition may sometimes be designated hereinafter as "lactic acid bacteria fermented sesame/soybean/Kombu" or "fermented sesame/soybean/Kombu"). The composition may be prepared by separately subjecting the starting materials Kombu, sesame and soybean (s) to lactic acid bacterial fermentation and then mixing the respective ferments together; alternatively, it may be prepared by mixing Kombu, sesame and soybean(s) together as the starting materials and inoculating the mixture with lactic acid bacteria.

In the case of fermenting the respective starting materials separately and thereafter mixing the ferments together to prepare the composition, namely, in the case of mixing the lactic acid bacteria fermented Kombu, the lactic acid bacteria fermented sesame and the lactic acid bacteria fermented soybean(s), the lactic acid bacteria fermented Kombu to be used can advantageously be prepared by, for example, the production method described above. The lactic acid bacteria fermented sesame to be used can be prepared in the same manner as the lactic acid bacteria fermented Kombu described above, or it may be the one described in JP 2004-173692 A, or the like. The lactic acid bacteria fermented soybean(s) to be used can be prepared in the same manner as the lactic acid bacteria fermented Kombu described above, or it may be the one described in JP 2003-335695 A, or the like.

The method of preparation by mixing Kombu, sesame and soybean(s) together as the starting materials and inoculating the mixture with lactic acid bacteria may be exemplified by the following procedure, to which the present invention is by no means limited.

First, Kombu, sesame and soybean(s) that serve as the starting materials for fermentation are provided. These starting materials are optionally processed into a powder or paste form. Alternatively, commercial materials in powder or paste form may be purchased and used as the starting materials for fermentation. In the next step, the starting materials Kombu (say, Kombu powder), soybean(s) (say, soybean flour) and sesame (say, sesame paste) are mixed at specified proportions, are added water, and are subjected to a sterilizing treatment. The temperature and time for the sterilizing treatment may be set as appropriate and it may be exemplified by one of holding at 90°C for 10 minutes. Subsequently, lactic acid bacteria is added to the resulting sterilized mixture (for example, a starter including lactic acid bacteria may be added) and a fermentation treatment is performed. In the case of performing the fermentation treatment, a saccharide such as glucose or a nitrogen source such as soybean peptide is preferably added as a fermentation promoting additive. The temperature and time for the fermentation treatment may be set as appropriate, depending on the nature of the lactic acid bacteria to be used. The degree of fermentation can be evaluated by measuring the pH of the mixture being subjected to the fermentation treatment. In the case of the composition of the present invention, fermentation is preferably continued until the pH becomes 5.0 or below. Note that if the mixture of the starting materials and water, or the sterilized mixture are unduly viscous in the step of sterilizing treatment and/or the step of fermentation treatment, an enzyme such as alginate lyase may be added to lower the viscosity of the mixture so that the operating efficiency is improved.

If the fermentation ends, the ferment is sterilized with heat, cooled, and optionally filtered. The temperature for sterilizing with heat may be set as appropriate. The thus obtained fermented composition in liquid form may be used as such, but from the viewpoint of ease in operation and storage, the liquid, fermented composition is preferably spray dried into a powder form. This powder is a brownish-red, fine powder, having pleasant smells that originate from the starting materials, and an acid taste that originates from the lactic acid bacteria.

It should be noted that, in any of the production processes, the lactic acid bacteria to be used can be chosen in the same manner as in the case of the lactic acid bacteria fermented Kombu, and it is preferred to use lactic acid bacteria of plant origin. The present inventors conducted preliminary tests using lactic acid bacteria isolated from Kyoto pickles and found that *Lactobacillus pentosus* and *Lactobacillus plantarum* could ferment each of Kombu, soybean(s) and sesame in a satisfactory way. Therefore, among the various lactic acid bacteria of plant origin, those lactic acid bacteria *(Lactobacillus pentosus* and *Lactobacillus plantarum)* can be used advantageously. In the production process in which Kombu, sesame and soybean(s) are mixed before inoculation with lactic acid bacteria, the above-mentioned *Lactobacillus pentosus* or *Lactobacillus plantarum* which can ferment each of Kombu, soybean(s) and sesame may be employed or, alternatively, a plurality of kinds of lactic acid bacteria that can ferment at least one of the materials may be employed simultaneously.

Immunomodulatory action

The composition of the present invention, namely, the lactic acid bacteria fermented Kombu or the lactic acid bacteria fermented sesame/soybean/Kombu is characterized by having an immunomodulatory action.

Immunity is a function that protect the body from an infinite number of pathogens or various harmful substances, to prevent various diseases, in other words, a function that maintains the homeostasis of the living body; an excessive working or disruption of immune function can cause allergic disease or autoimmune disease. The immunomodulatory action as referred to in the present invention means an action of stimulating an immune reaction when part of the immune function is disrupted or the immune function is weakened due to aging, stress, fatigue, lack of sleep, etc. (this action is called an immunostimulating action), or an action of suppressing an immune reaction when the immune function works excessively (this action is called an immuno-suppressing action); the composition of the present invention intends to optimize the immune balance by allowing the immunostimulating action and the immuno-suppressing action to function properly.

The immune system in the living body includes lymphocytes, T cells that recognize antigens; and they are classified into Th1 cells and Th2 cells according to the cytokines they produce. The balance between Th1 and Th2 (Th1/Th2 balance) affects immune reactions in the living body; speaking of allergy, for example, it is known that if Th1 becomes dominant, delayed hypersensitivity (type IV allergy such as rejection that occurs in organ transplantation) is induced, and that if Th2 becomes dominant, an antigen-specific IgE antibody is produced, inducing immediate hypersensitivity (type I allergy such as asthma, allergic rhinitis, pollinosis, or atopic dermatitis). Th1 cells produce IFN-$\gamma$ whereas Th2 cells produce IL-4; hence, the ratio between IL-4 and IFN-$\gamma$ (IFN-$\gamma$/IL-4) can be used as an index of the immune balance.

The composition of the present invention (the lactic acid bacteria fermented Kombu and the lactic acid bacteria fermented sesame/soybean/Kombu) is characterized by bringing the Th1/Th2 balance in the living body to a Thl-dominant state. As mentioned above, it is known that Th2 is dominant in type I allergy such as asthma, pollinosis, or atopic dermatitis. In addition, it has been reported that Th2 is also dominant in HIV infection. Further, it is known that if Th1 becomes dominant, the activity of macrophages, NK cells or cytotoxic T lymphocytes (CTL) is promoted to increase resistibility against cancer. Therefore, by ingesting the composition of the present invention either on its own or as food or medicament so that Th1 becomes dominant, not only can type I allergy be treated or prevented but also the resistibility to HIV, cancer or bacterial infection can be enhanced to allow for their prevention or treatment.

The composition of the present invention is also characterized by the ability to improve the immune strength of the intestinal tract. Intestinal tract immunity is the defense system that exists on the surface of the mucous membrane of the intestinal tract to ensure that any pathogenic factors getting into the intestinal tract will be eliminated either through the nose or throat or together with food or beverage. The control tower in this system is Peyer's patch cells distributed on the mucous membrane of the small intestine, and it is known that the antibody (IgA) released from Peyer's patch cells attacks pathogenic microbes, viruses and other foreign matter.

Thus, the composition of the present invention (the lactic acid bacteria fermented Kombu or the lactic acid bacteria fermented sesame/soybean/Kombu) is useful as an immunomodulatory, immunostimulating, immuno-suppressing or anti-allergic composition.

Food/beverage, medicament, etc.

The composition of the present invention can be used as food/beverage, medicament, etc.; however, since this composition is accomplished by adding an immunomodulatory function to common foodstuffs of high nutritional value, or by enhancing their immunomodulatory function, and features high safety, it is preferably used as food or beverage that can be ingested on a daily basis. If it is to be used in food or beverage, it is advantageously applied as health food having an immunomodulatory action. Examples of the health food as referred to herein also include foods and beverages that have an indication on the container or instruction manual, stating that they have an immunomodulatory action, an immunostimulating action and/or an anti-allergic action (for example, functional foods such as foods for specified health use and qualified foods for specified health use). The place where such statement is indicated (e.g. container or instruction manual affixed on it), the type of container (e.g. bottle, can, PET bottle, plastic bottle, paper pack, etc.), and the method of indication (e.g., printing, stamping, seals, etc.) are not limited in any way. It should be noted here that modes of use as food also include the case where the composition of the present invention is used as a food additive.

If the composition of the present invention is to be used as food or beverage, it may be used as such or, if desired, it may be mixed with a variety of ingredients such as known bases, aids, sweeteners, sour agents, vitamins, etc. to formulate products that suit the preference of users; for example, the composition of the present invention can be offered in various forms such as tablet, capsule, pill, powder, granule, candy, drop, troche, gum, powdered juice, health drink, seasoning, processed food, dessert, and confection. In addition, since the lactic acid bacteria fermented Kombu or the lactic acid bacteria fermented sesame/soybean/Kombu of the present invention tastes mild, one of the advantageous forms is such that the taste can be appreciated by chewing the food or beverage. Specific examples that can be mentioned are a form such as pill and a form such as biscuit. Pills are usually conditioned to have an oil content of 1% or less. This is to prevent pills from being degraded by oxidation or sticking together, each on account of the exudation of oil during storage. If defatted soybean(s) and sesame are used in the lactic acid bacteria fermented sesame/soybean/Kombu of the present invention, the oil content of a pill can be adjusted to 1% or less; however, considering the nutritional value of the foodstuffs (particularly sesame), it is not necessarily appropriate to use defatted ingredients. If defatted ingredients are not used, oil is contained in amounts of about 4-15%, causing the above-mentioned problem of oil exudation. The present inventors made studies on a process for producing pills that are protected against this problem of oil exudation; as a result, they found that exudation of oil is marked in the step of uncoated pill buffing (a treatment for buffing the surface of a uncoated pill) which is usually applied to provide a better appearance; the inventors then confirmed that the exudation of oil could be prevented by coating the uncoated pill with shellac or sugar without performing the step of uncoated pill buffing.

The food or beverage of the present invention can also be used in animals other than humans for the purpose of modulating the immune function (e.g. immunostimulation, anti-allergy, etc.). Animals that can be treated include, for example, pets such as dog and cat, farm animals such as pig and cattle, poultry such as chicken, laboratory animals such as mouse and guinea pig, as well as cultured marine animals. As for the form of the food and beverage, they can be used on their own but, if desired, they can be added to feed (pet foods) for animals or fishery products, or can be provided in a form such as supplements.

Use as medicaments may be exemplified by immunomodulators such as an immunostimulating agent and an anti-allergic agent. In addition, the composition of the present invention can be formulated as pharmaceutical preparations by combining the active ingredient with known aids that are commonly used in the art of pharmaceutical formulation procedures, such as excipients, binders, disintegrants, lubricants, flavoring/odorizing agents, solvent promoters, suspending agents, coating agents, etc. Dosage forms include, but are not limited in any particular way to, pill, tablet, capsule, granule, powder, syrup, suppository, injection, etc. The duration of administration is not limited in any particular way, but continuous administration for one week to 10 days or more is recommended, with continuous administration on a permanent basis being preferable.

The amount in which the composition of the present invention (the lactic acid bacteria fermented Kombu or the lactic acid bacteria fermented sesame/soybean/Kombu) is contained in the food/beverage or medicament of the present invention is not limited in any particular way, but it is generally on the order between 0.1 and 100 wt%, preferably between 1 and 90%, more preferably between 1 and 50 wt%.

The food/beverage or medicament that consists of, or comprises, the composition of the present invention contains in a single dosage the lactic acid bacteria fermented Kombu or the lactic acid bacteria fermented sesame/soybean/Kombu in an amount of 1-10000 mg, preferably 10-10000 mg, in terms of dry weight; however, since the composition of the present invention features high safety, the upper limit of its content is substantially absent.

EXAMPLES

On the pages that follow, the composition of the present invention is described in detail by reference to examples. It should, however, be noted that the present invention is by no means limited to those examples.

Test 1 Lactic Acid Bacterial Fermentation Test (1)

Kombu was investigated for the possibility of fermentation by lactic acid bacteria. The Kombu used was in a powder form (trade name "KOMBU POWDER" produced by Yaizu Suisankagaku Industry Co., Ltd.). To this Kombu, distilled water was added such that the ratio of Kombu:water would be 5:95 (on weight basis), and the two components were mixed together; the resulting mixture in liquid form was sterilized at 110 °C for one minute. Thereafter, one of the eight lactic acid bacteria listed in Table 1 (A-H) (see JP 2005-333919 A) was inoculated as a starter to give a concentration of 0.5%. The resulting mixtures were fermented at 37 °C for 48 hours, and pH measurement was done before and after the fermentation; as for lactic acid bacteria A-D, the number of cells after fermentation was counted. Note that lactic acid bacteria A (S-PT84) mentioned in the table has been deposited with the International Patent Organism Depositary, National Institute of Advanced Industrial Science and Technology, under accession number FERM ABP-10028.
[Table 1]

|   | Strain | Species | Extracellular polysaccharide (EPS) |
|---|--------|---------|-----------------------------------|
| A | DS84C (S-PT84) | Lactobacillus pentosus | + |
| B | DS84N | Lactobacillus pentosus | - |
| C | DW69C | Lactobacillus pentosus | + |
| D | DW69N | Lactobacillus pentosus | - |
| E | DB22C | Lactobacillus plantarum | + |
| F | DB22N | Lactobacillus plantarum | - |
| G | DS2C | Lactobacillus plantarum | + |
| H | DS2N | Lactobacillus plantarum | - |

The results for the cell count after fermentation and the pH before and after fermentation are shown in Table 2. Whichever of the eight lactic acid bacteria was used, an increase in the lactic acid bacterial cell count and/or a decrease in pH was confirmed, thus making it clear that the lactic acid bacteria cause the fermentation of Kombu to proceed.
[Table 2]

|   | A | B | C | D | E | F | G | H |
|---|---|---|---|---|---|---|---|---|
| $10^7$cuf/g | 2 | 3 | 6 | 3 | - | - | - | - |
| $10^5$cuf/g | 235 | 116 | >300 | >300 | - | - | - | - |
| Initial pH | 5.83 | | | | | | | |
| Final pH | 4.78 | 4.59 | 4.33 | 4.52 | 4.55 | 5.38 | 4.80 | 5.01 |

Test 2 Lactic Acid Bacterial Fermentation Test (2)

Sesame and soybeans were investigated for the possibility of fermentation by the lactic acid bacteria used in Test 1. The sesame used was in a paste form (trade name "JUN-NERI GOMA KURO" produced by TAKEMOTO OIL & FAT Co., Ltd.). and the soybeans used was in a powder form ("KURODAIZU KINAKO" produced by K. Kobayashi Co., Ltd.). To these, distilled water was added such that the respective ratios of sesame:water and soybean:water would be 25:75 and 15:85 (both on weight basis) and the two components were mixed together; sterilizing and fermentation treatment were performed as in Test 1; pH measurement was done before and after the fermentation, and the number of cells after fermentation was counted. Note that cell counting after fermentation was done for the eight lactic acid bacteria on sesame, and for lactic acid bacteria A-D on soybean(s).
The results for the cell count after fermentation, and the pH before and after fermentation are shown in Table 3. Whichever of the eight lactic acid bacteria was used, an increase in the lactic acid bacterial cell count and/or a decrease in pH was confirmed, thus making it clear that the lactic acid bacteria cause the fermentation of sesame and soybean (s) to proceed.

[Table 3]

|  |  | A | B | C | D | E | F | G | H |
|---|---|---|---|---|---|---|---|---|---|
| Sesame | $10^7$cuf/g | 30 | 73 | 28 | 40 | 33 | 15 | 5 | 5 |
|  | Initial pH | 6.13 | | | | | | | |
|  | Final pH | 4.58 | 4.50 | 4.67 | 4.68 | 4.77 | 4.74 | 5.22 | 5.16 |
| Soybean | $10^7$cuf/g | 73 | 56 | 92 | 106 | - | - | - | - |
|  | Initial pH | 6.08 | | | | | | | |
|  | Final pH | 4.36 | 4.33 | 4.51 | 4.54 | 4.53 | 4.28 | 4.54 | 4.24 |

<u>Example 1</u> Preparation of Lactic Acid Bacteria Fermented Kombu

Kombu in a powder form (trade name "KOMBU POWDER" produced by Yaizu Suisankagaku Industry Co., Ltd.) was used (this is hereinafter designated as "unfermented Kombu"). To the unfermented Kombu, distilled water having alginate lyase (product of Nagase Chemtex corporation) dispersed in it was added and the mixture was sterilized with heat at 90 °C for 20 minutes. The starter used was a bulk starter obtained by culturing lactic acid bacteria *(Lactobacillus pentosus* strain S-PT84) under the nutrient conditions of 2% glucose, 1% yeast extract (Mitsubishi-Kagaku Foods Corporation) and 1% soybean peptide (product of FUJI OIL CO., LTD.) for 20 hours. This starter was added to the Kombu mixture in liquid form that had been subjected to sterilizing treatment, and fermentation was performed at a temperature of about 37 °C for about 14 hours. After the end of fermentation, the fermentation liquor was sterilized with heat at 90 °C for 10 minutes, cooled, and filtered through 20 mesh; the resulting filtrate was dried with a spray dryer (Mini-Spray Dryer Model B-290, product of Nihon BUCHI K.K.) to yield a lactic acid bacteria fermented Kombu composition (which is hereinafter designated as "fermented Kombu").

<u>Example 2</u> Preparation of Lactic Acid Bacteria Fermented Sesame, Soybean and Kombu in Mixture (Sesame/Soybean/Kombu)

The sesame used was "JUN-NERI GOMA KURO" produced by TAKEMOTO OIL & FAT Co., Ltd.; the soybean (s) used was "KURODAIZU KINAKO" produced by K. Kobayashi Co., Ltd.; and as in Example 1, the Kombu used was "KOMBU POWDER" produced by Yaizu Suisankagaku Industry Co., Ltd.) that had been treated with alginate lyase produced by Nagase Chemtex corporation. The sesame, soybean(s) and Kombu were mixed to give a ratio of 1:3:1 (by weight) (the mixture is hereinafter designated as "unfermented sesame/soybean/Kombu" or "unfermented composition"); to this mixture, muscovado (at a weight ratio of 1/3 to the sesame) and soybean peptide (at a weight ratio of 1/15 to the sesame) were added as fermentation promoting additives. The resulting sesame, soybean(s) and Kombu in mixture was fermented as in Example 1; thereafter, the ferment was sterilized with heat at 90 °C for 10 minutes, cooled, and filtered through 20 mesh; the resulting filtrate was dried with a spray dryer (Mini-Spray Dryer Model B-290, product of Nihon BUCHI K.K.) to yield a lactic acid bacteria fermented composition of sesame, soybean(s) and Kombu in mixture (which is hereinafter designated as "fermented sesame/soybean/Kombu" or "fermented composition").

<u>Example 3</u> Promoting Action on IL-12 Production from Macrophages (In vitro Test)

C57BL/6 mice (7-week old, male, 10 animals, from SHIMIZU Laboratory Supplies Co., Ltd.) were administered intraperitoneally with 5 mL of PBS (Phosphate-Buffer Salines) per animal and intraperitoneal cells were recovered aseptically. The recovered cells were washed with a cell culture medium (10% PBS (fetal bovine serum) supplemented RPMI 1649 medium (NISSUI PHARMACEUTICAL CO., LTD.)) and erythrocytes were removed by hemolysis with a hemolytic buffer. To be more specific, 1 mL of a hemolytic buffer (155 mM $NH_4Cl$, 10 mM $KHCO_3$, and 0.1 mM EDTA) was added to the recovered cells, which were left to stand for 2-3 minutes at room temperature, and 9 mL of a cell culture medium was added. After stirring, the mixture was centrifuged (20 °C, 1500 rpm, 3 min) and the precipitate was washed once with a cell culture medium. Using a blood cell counter, the number of cells was counted and a solution having a concentration of 1 x $10^6$ cells/mL was prepared in a cell culture medium; the resulting solution was sown on a 48-well culture plate (tissue culture plate) such that each well contained 500 $\mu$L of the solution. After 3-hr culture in 5% $CO_2$ at 37 °C, the medium was removed to remove the non-adherent cells and a cell culture medium was added again.

The unfermented Kombu, fermented Kombu and fermented sesame/soybean/Kombu that were obtained in Examples 1 and 2 were weighed in specified amounts, ground in a mortar and suspended in a cell culture medium to prepare liquid samples (suspension of the unfermented Kombu, suspension of the fermented Kombu, and suspension of the fermented sesame/soybean/Kombu); these samples were added to the above-described cell plate in such amounts as to give final concentrations of 1 $\mu$g/mL and 10 $\mu$g/mL and cultured in 5% $CO_2$ at 37 °C for 18 hours. As a comparison, a suspension of S-PT84 (dry dead cells) in a cell culture medium was added in such amounts as to give final concentrations of 1.5 ng/mL and 15 ng/mL and cultured in 5% $CO_2$ at 37 °C for 18 hours. Note that the final concentrations of strain S-PT84 corresponded to the amounts of strain S-PT84 in the suspensions of fermented sesame/soybean/Kombu at 1 $\mu$g/mL and 10 $\mu$g/mL. The amount of cytokine (interleukin 12: IL-12) contained in the culture supernatant was measured with an ELISA kit (OptEIA, BD Pharmingen). In addition, a culture solution to which nothing had been added was used as a control (Cont) and subjected to similar measurement.

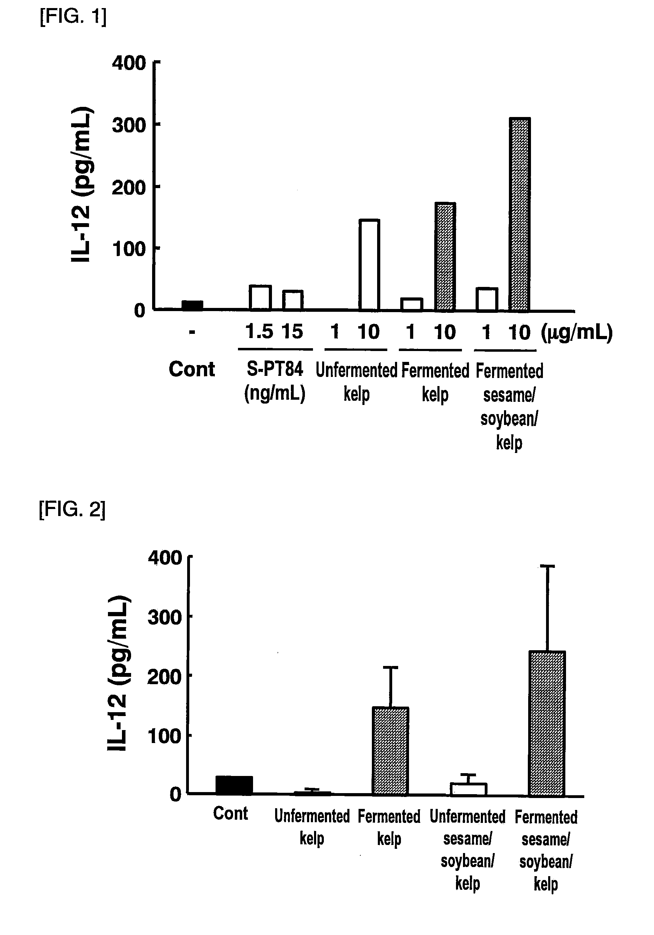

The test results for the IL-12 concentration in the culture supernatant (the average for N=2) are shown in FIG. 1. The addition of the unfermented Kombu, fermented Kombu and fermented sesame/soybean/Kombu at the higher concentrations caused distinct increases in the IL-12 concentration in the culture solution. In addition, it was suggested that the addition of the fermented Kombu increased the IL-12 concentration more than in the case where the unfermented Kombu was added and further that the addition of the fermented sesame/soybean/Kombu increased the IL-12 concentration more than in the case where the fermented Kombu was added. From these results, it became clear that the fermented Kombu had a stronger immunostimulating action than the unfermented Kombu and that the immunostimulating action of the fermented sesame/soybean/Kombu was greater than that of the fermented Kombu.

Example 4 Promoting Action on IL-12 Production from Macrophages (Ex vivo Test)

C57BL/6 mice (7-week old, male, 15 animals) were divided into five groups each consisting of 3 animals. These groups were as follows: an untreated group (Control), a group administered with the unfermented Kombu (10 mg/mouse), a group administered with the fermented Kombu (10 mg/mouse), a group administered with the unfermented sesame/soybean/Kombu (10 mg/mouse), and a group administered with the fermented sesame/soybean/Kombu (10 mg/mouse). In the groups excepting Control, PBS was added to the unfermented Kombu, fermented Kombu, unfermented sesame/soybean/Kombu, and fermented sesame/soybean/Kombu that had been obtained in Examples 1 and 2, so as to prepare suspensions that contained them at a concentration of 20 mg/mL; the resulting suspensions were each administered intraperitoneally in an amount of 0.5 mL. Eighteen hours after the administration, 5 mL of PBS was administered intraperitoneally and intraperitoneal cells were recovered aseptically. The recovered cells were washed with a cell culture medium and erythrocytes were removed by hemolysis with a hemolytic buffer as in Example 3. Using a blood cell counter, the number of cells was counted and a solution having a concentration of $2 \times 10^6$ cells/mL was prepared using a cell culture medium; the resulting solution was sown on a 48-well culture plate (tissue culture plate) such that each well contained 500 $\mu$L of the solution. After 3-hr culture in 5% $CO_2$ at 37 °C, non-adherent cells were removed and LPS (lipopolysaccharide) was added to give a final concentration of 100 ng/mL, followed by culture in 5% $CO_2$ at 37 °C for 24 hours. The amount of IL-12 contained in the culture supernatant was measured with an ELISA kit (OptEIA, BD Pharmingen).

The results of measurement of the IL-2 concentration in the culture supernatant (the average for N=2-3) are shown in FIG. 2. As is clear from these results, no increase in the IL-12 concentration was recognized in the group administered with the unfermented Kombu and in the group administered with the unfermented sesame/soybean/Kombu; however, a large increase in the IL-12 production was recognized in the group administered with the fermented Kombu and in the group administered with the fermented sesame/soybean/Kombu, and particularly in the group administered with the fermented sesame/soybean/Kombu, the amount of IL-12 production increased significantly. From these results, it became clear that neither the unfermented Kombu nor the unfermented sesame/soybean/Kombu was effective in potentiating the action of macrophages by LPS stimulation but that such effectiveness was found in the fermented Kombu and the fermented sesame/soybean/Kombu, with the fermented sesame/soybean/Kombu being more effective than the fermented Kombu.

The results of Examples 3 and 4 show that the composition of the present invention has superior immunomodulatory actions that can not be expected from the starting materials that were fermented to make that composition.

Example 5 Effects of Kombu Ferment on NK Activity and on Balance Between Th1 Cells and Th2 Cells (Th1/Th2)

Twelve C57BL/6 mice (6-week old, male) were divided into four groups, each consisting of 3 animals and having almost the same average weight. These groups were as follows: an untreated group (Control), a group administered with S-PT84, a group administered with the unfermented Kombu, and a group administered with the fermented Kombu. As for the groups excepting the untreated group, S-PT84 (dry dead cells) was forcibly administered orally daily in an amount of 0.75 mg/kg per head (S-PT84 administered group); and so were the unfermented Kombu of Example 1 (in the unfermented Kombu administered group), and the fermented Kombu of Example 1 (in the fermented Kombu administered group), except that the amount was 500 mg/kg per head. Note that the amount of S-PT84 in the S-PT84 administered group corresponds to the amount of S-PT84 in the fermented sesame/soybean/Kombu. At day 7 after the start of administration, the spleen was extracted aseptically and lightly compressed on a cell strainer (70 $\mu$m, product of BD Falcon) in a cell culture medium (10% fetal bovine serum, 0.2% antibiotic-antifungal agent mixed solution (product of Nakalai Tesque)), to recover the cells. Dispersing the cells by pipetting was followed by centrifugation (1500 rpm, 3 min, 4 °C). As in Example 3, erythrocytes were removed by hemolysis and spleen lymphocytes were then prepared. The NK activity of the spleen lymphocytes was measured by the PINK method (in the PINK method, target cells Yac-1 are labeled with the hydrophobic membrane-based fluorochrome 3,3'-dioctadecycloxacarbocyanine perchlorate (Dio) whereas the nuclei of dead cells are double-stained with the membrane-impermeable, nucleic acid binding fluorochrome propidium iodide (PI), and the intact Yac-1 which is single-stained with Dio, and the damaged Yac-1 which is double-stained are detected by flow cytometry, whereby the cytotoxic activity of the mouse spleen cells is computed). The spleen lymphocytes were also cultured for 24 hours under stimulation with concanavalin A (2.5 $\mu$g/mL), and the amounts of IFN-$\gamma$ and IL-4 that were produced in the culture supernatant were measured by ELISA with OptEIA (BD Pharmingen).

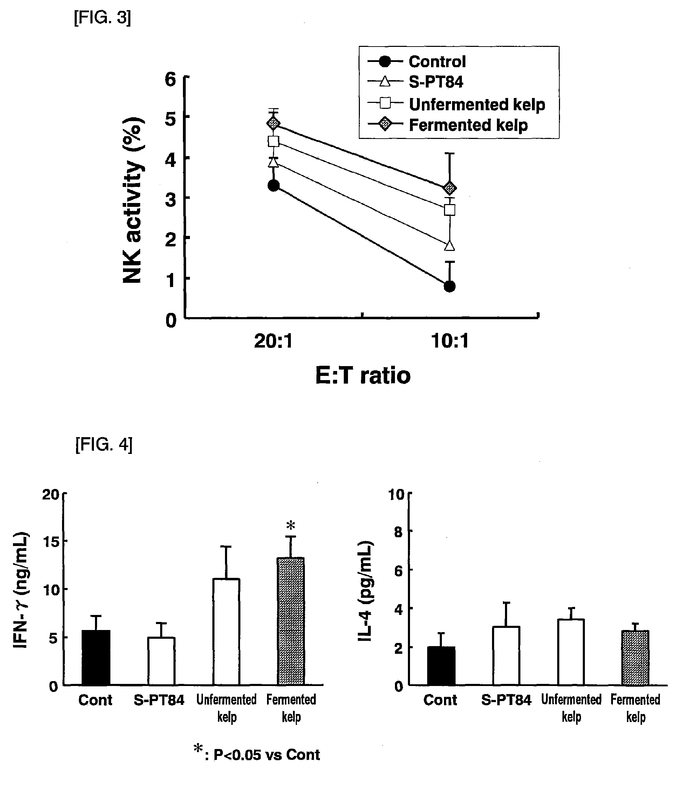

The results for the NK activity are shown in FIG. 3. In FIG. 3, NK activity (%) shows the cytotoxicity of mouse spleen lymphocytes on Yac-1 (this is mouse lymphoma derived cells which are normally used as cells sensitive to mouse NK cells; the results are data at mouse spleen lymphocyte to Yac-1 ratios of 20:1 and 10:1). As is clear from FIG. 3, the NK activity which was the lowest in the control group increased in the S-PT84 administered group, the unfermented Kombu administered group and the Kombu ferment administered group in the order written.

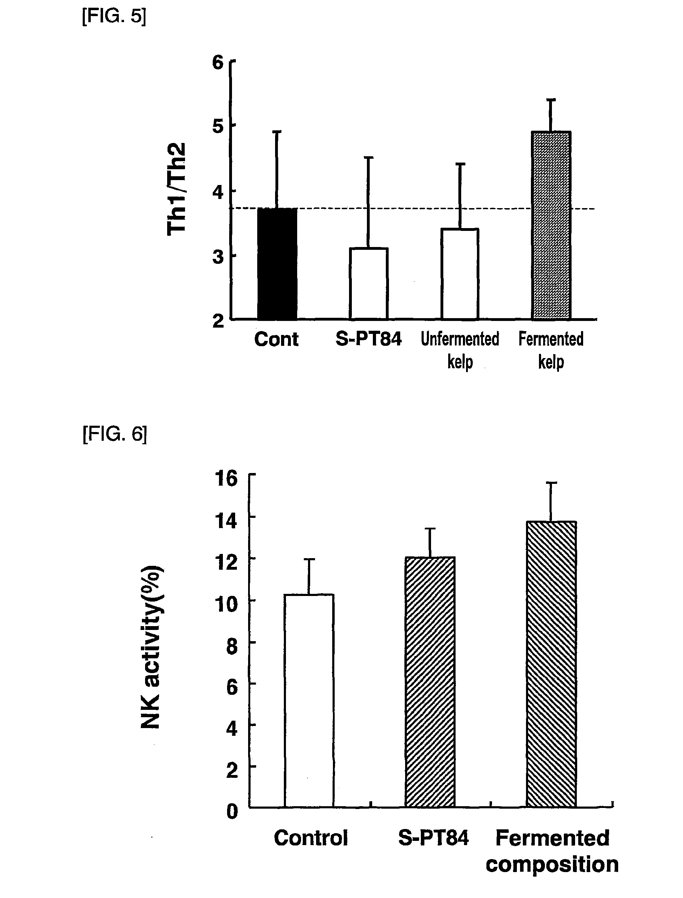

The results for IFN-$\gamma$ concentration and IL-4 concentration are shown in FIG. 4, whereas that for Th1/Th2 (IFN-$\gamma$/IL-4), which is an index for the immune balance, is shown in FIG. 5. As is clear from FIG. 4, the IFN-$\gamma$ concentration did not change in the S-PT84 group as compared with the control group, but it increased in the unfermented Kombu administered group, and increased significantly in the Kombu ferment administered group. As for the IL-4 concentration, the production was a little higher in all of the administered groups as compared with the control group, but the increase was only 40-70%. According to FIG. 5 which was constructed on the basis of these results, the Th1/Th2 balance in the S-PT84 administered group and the unfermented Kombu administered group was not different from the value in the control group, and it was only in the fermented Kombu administered group that was in a Th1 dominant state. From these results, it became clear that the fermented Kombu is effective in potentiating the immune function in a steady state, and that it also has an action of adjusting the Th1/Th2 balance to a Th1 dominant state.

Example 6 Effects of Fermented Sesame/Soybean/Kombu on NK Activity and on Balance Between Th1 Cells and Th2 Cells (Th1/Th2)

Eighteen C57BL/6 mice (6-week old, male) were divided into six groups, each consisting of 6 animals and having almost the same average weight. These groups were as follows: an untreated group (Control), an S-PT84 ingesting group (S-PT84), and a group ingesting the fermented sesame/soybean/Kombu obtained in Example 2 (Fermented Composition). Each of the ingesting groups were allowed to ingest ad libitum for a week a solid feed to which S-PT84 (dry dead cells) had been added in an amount of 0.0075 wt%, or a solid feed to which the fermented sesame/soybean/Kombu had been added in an amount of 5 wt%. Note that the amount of S-PT84 in the S-PT84 ingesting group corresponds to the amount of S-PT84 in the fermented sesame/soybean/Kombu. At day 8 after the start of ingestion, the spleen was extracted and spleen lymphocytes were prepared as in Example 5. The NK activity of the obtained spleen lymphocytes (PINK method) and the IL-4 and IFN-$\gamma$ concentrations under stimulation with concanavalin A (2.5 $\mu$g/mL, 24 hours) were measured as in Example 5.

The results for the NK activity are shown in FIG. 6. In FIG. 6, NK activity (%) shows the cytotoxicity of mouse spleen lymphocytes on Yac-1 (this is mouse lymphoma derived cells which are normally used as cells sensitive to mouse NK cells; the results are data at a mouse spleen lymphocyte to Yac-1 ratio of 80:1). As is clear from FIG. 6, the NK activity increased in the S-PT84 ingesting group as compared with the untreated group, and the increase was more marked in the fermented sesame/soybean/Kombu ingesting group. Therefore, the composition of the present invention was shown to have an immunostimulating action.

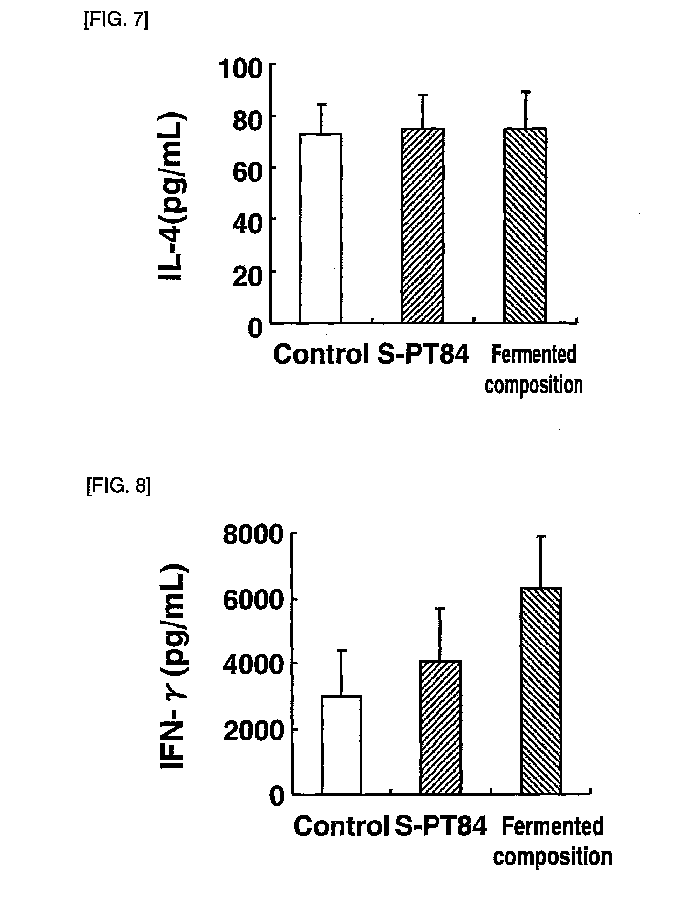

The results for IL-4 concentration and IFN-$\gamma$ concentration are shown in FIGS. 7 and 8, respectively. As is clear from FIG. 7, there was no appreciable change in the IL-4 concentration caused by ingesting S-PT84 or the fermented sesame/soybean/Kombu. On the other hand, according to FIG. 8 which refers to the IFN-$\gamma$ concentration, it increased in the S-PT84 group as compared with the untreated group, and the increase was more pronounced in the fermented sesame/soybean/Kombu ingesting group.

Thus, it became clear from Example 6 that the composition of the present invention changes the Th1/Th2 balance to a Th1 dominant state.

Example 7 Effects of Fermented Sesame/Soybean/Kombu on Stress-Induced Immune Compromise and on Balance Between Th1 Cells and Th2 Cells (Th1/Th2)

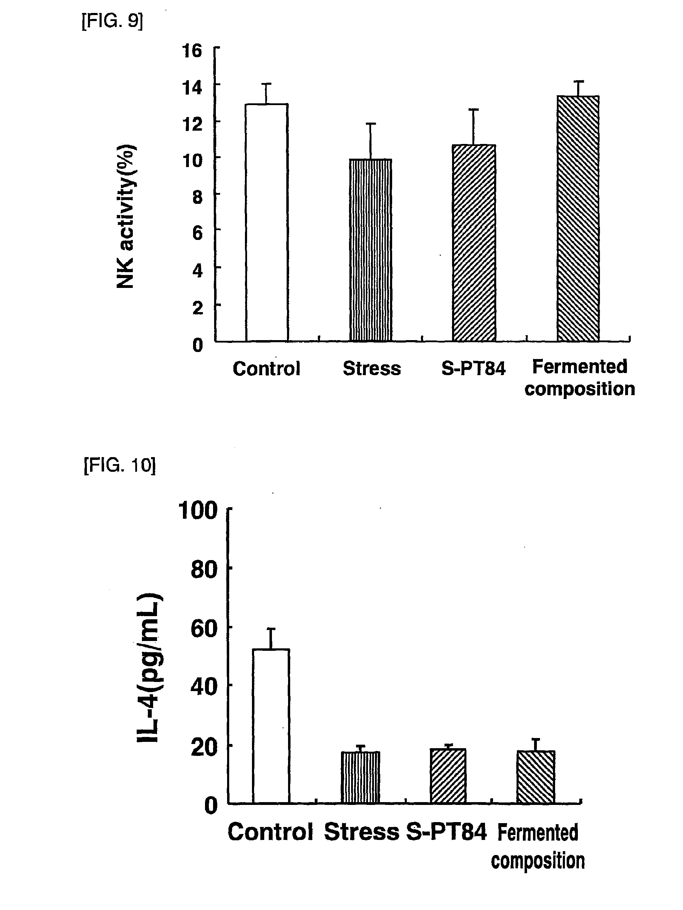

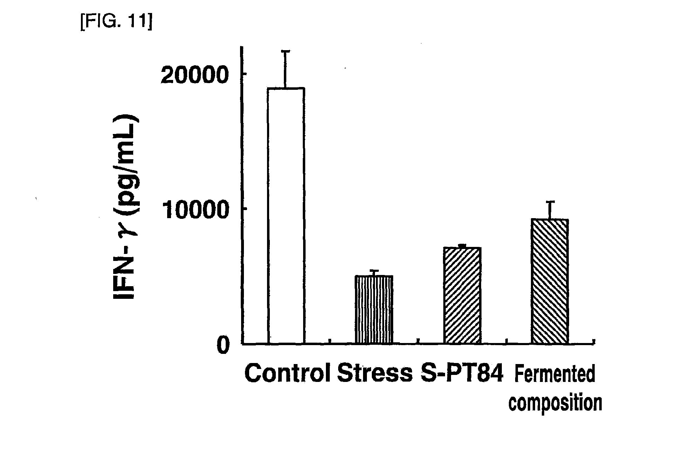

Twenty-four C57BL/6 mice (6-week old, male) were divided into four groups, each consisting of 6 animals and having almost the same average weight. These groups were as follows: an untreated group (Control), a stress applied group (Stress), a stress applied + S-PT84 ingesting group (S-PT84), and a group placed under stress and allowed to ingest the fermented sesame/soybean/Kombu obtained in Example 2 (Fermented Composition). Each of the ingesting groups were allowed to ingest ad libitum for a week a solid feed to which S-PT84 (dry dead cells) had been added in an amount of 0.0075 wt% or a solid feed to which the fermented sesame/soybean/Kombu had been added in an amount of 5 wt%. Note that the amount of S-PT84 in the S-PT84 ingesting group corresponds to the amount of S-PT84 in the fermented sesame/soybean/Kombu. At day 8 after the start of ingestion, the 18 animals under stress were submerged once in water, put into 50-mL polyethylene tubes having an air hole opened at an end, and restrained for 24 hours. The untreated group was kept from water and meal for 24 hours. Thereafter, the spleen was extracted and spleen lymphocytes were prepared as in Example 5 for measurement of the NK activity (PINK method). In addition, the IL-4 and IFN-y concentrations under stimulation with concanavalin A (2.5 $\mu$g/mL, 24 hours) were measured.
The results for the NK activity are shown in FIG. 9. In FIG. 9, NK activity (%) shows the cytotoxicity of mouse spleen lymphocytes on Yac-1. As is clear from FIG. 9, the NK activity decreased in the stress applied group (Stress) as compared with the untreated group. In the S-PT84 ingesting group, the lowering of NK activity due to stress was suppressed, and in the fermented sesame/soybean/Kombu ingesting group, the NK activity was maintained at the level in the untreated group. The results for IL-4 concentration and IFN-$\gamma$ concentration are shown in FIGS. 10 and 11. As is clear from FIGS. 10 and 11, the application of stress caused a marked decrease in both IL-4 and IFN-$\gamma$ concentrations (Stress). In that group, the IFN-$\gamma$ concentration decreased more markedly than the IL-4 concentration, so it is understood that this changed the Th1/Th2 balance to a Th2 dominant state. There was no appreciable change in the IL-4 concentration under stress, caused by ingesting S-PT84 or the fermented sesame/soybean/Kombu. On the other hand, the IFN-$\gamma$ concentration increased in the S-PT84 ingesting group as compared with the stress applied group, and the increase was more pronounced in the fermented sesame/soybean/Kombu ingesting group.
From the foregoing results, it became clear that the product obtained by fermenting sesame/soybean/Kombu with lactic acid bacteria (fermented sesame/soybean/Kombu) not only potentiates the immune function in a steady state but also suppresses the immune compromise under stress. These effects were recognized markedly in the fermented sesame/soybean/Kombu, but were not recognized markedly in the lactic acid bacteria alone.

Example 8 Anti-allergic Action of Fermented Sesame/Soybean/Kombu

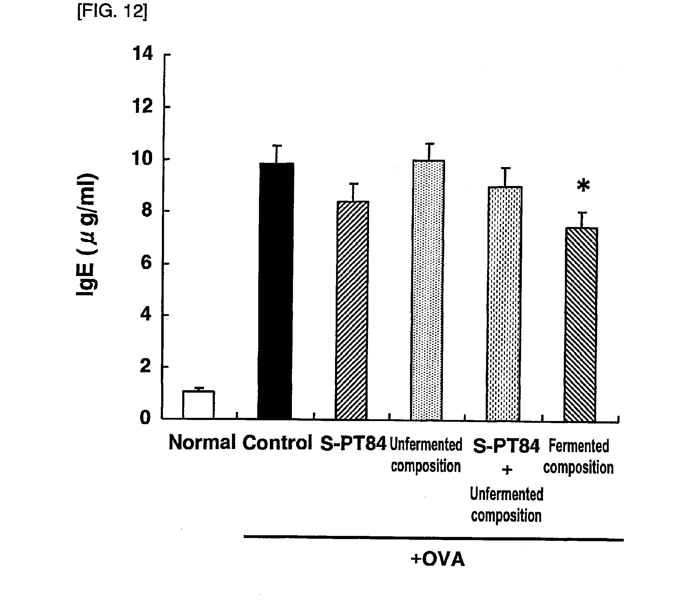

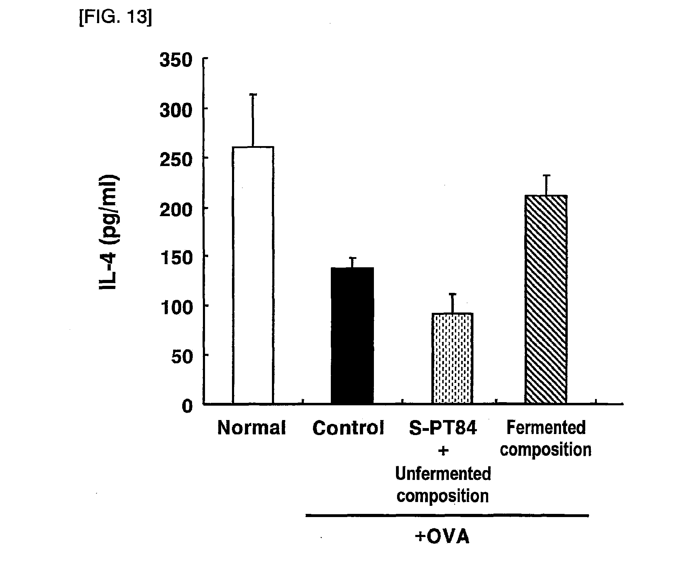

BALB/C mice (7-week old, male) were divided into six groups. These groups were as follows: i) an untreated group of 3 animals (Normal), ii) a control group of 9 animals (Control), iii) an S-PT84 (dry dead cells mixed in 0.0075%) ingesting group of 12 animals, iv) a group ingesting the unfermented sesame/soybean/Kombu obtained in Example 2 (mixed in 5%) of 9 animals (Unfermented Composition), v) a group ingesting S-PT84 (mixed in 0.0075%) + the unfermented sesame/soybean/Kombu (mixed in 5%) of 10 animals, and vi) a group ingesting the fermented sesame/soybean/Kombu obtained in Example 2 (mixed in 5%) of 8 animals (Fermented Composition). To the S-PT84 group and the group ingesting S-PT84 + the unfermented sesame/soybean/Kombu, S-PT84 was added to a solid feed in a cell count that corresponded to the number of cells contained in the fermented sesame/soybean/Kombu. The untreated group and the control group were allowed to ingest ad libitum a basic feed of AIN-93N (Oriental Bioservice Inc.); and the other groups were allowed to ingest ad libitum a special feed prepared by mixing AIN-93N with the ingredients specified above. One week and two weeks after the start of ingestion, 20 $\mu$g of ovalbumin (OVA) and 2 mg of aluminum hydroxide gel were mixed and administered intraperitoneally into the mice in the five groups excluding the untreated group. Four weeks after the start of ingestion, blood was taken from the heart, and serum samples were prepared for measuring the total IgE concentration in the serum. At the same time, spleen cells were separated and cultured, and following 24-hr culture under stimulation with concanavalin A (2.5 $\mu$g/mL), and the amounts of INF-$\gamma$ and IL-4 produced in the culture supernatant were measured. Measurements of total IgE, INF-$\gamma$ and IL-4 were performed by ELISA with OptEIA (BD Pharmingen). It should be noted that the total intake of the feed by each of the animal groups was about 3.0 g.
The test results for the total IgE concentration are shown in FIG. 12. As is clear from FIG. 12, it was only in the group ingesting the fermented sesame/soybean/Kombu of the present invention that the elevation of IgE was suppressed significantly.
The spleen cells from each group were stimulated with concanavalin A, and the amount of subsequent IL-4 production is shown in FIG. 13. By OVA administration, the amount of IL-4 production (in the control group) decreased as compared with the untreated group. The amount of IL-4 production further decreased in the S-PT84 + unfermented sesame/soybean/Kombu ingesting group. However, in the fermented sesame/soybean/Kombu ingesting group, the amount of IL-4 production increased as compared with the control group.

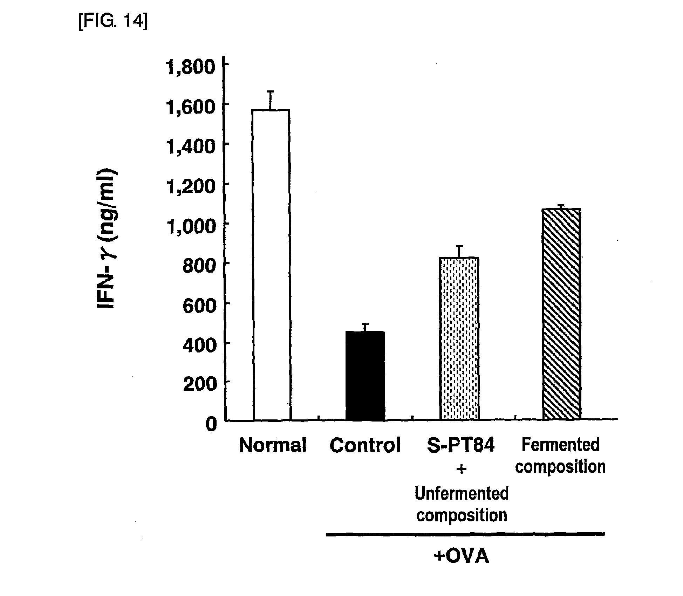

The spleen cells from each group were stimulated with concanavalin A and the amount of subsequent IFN-γ production is shown in FIG. 14. Upon OVA administration, the amount of IFN-γ production (in the control group) decreased as compared with the untreated group. However, in the S-PT84 + unfermented sesame/soybean/Kombu ingesting group and the fermented sesame/soybean/Kombu ingesting group, the amount of IFN-γ production increased as compared with the control group. This effect was particularly pronounced in the fermented sesame/soybean/Kombu ingesting group.

From the results of Example 8, it became clear that the fermented sesame/soybean/Kombu of the present invention has an anti-allergic action. However, no allergic action was recognized from the ingestion of the unfermented sesame/soybean/Kombu or the unfermented sesame/soybean/Kombu + lactic acid bacteria. Therefore, it became clear that fermenting sesame/soybean/Kombu with lactic acid bacteria is important for exhibiting the anti-allergic action.

Example 9 IgA Secretion Enhancing Action by Oral Ingestion of Fermented Sesame/Soybean/Kombu

C57BL/6 mice (7-week old, male) were divided into three groups such that each group had almost the same average weight. These groups were as follows: a control group of 7 animals (Control), an S-PT84 (dry dead cells mixed in 0.0075%) ingesting group of 9 animals, and a group ingesting the fermented sesame/soybean/Kombu obtained in Example 2 (mixed in 5%) of 9 animals (Fermented Composition). In S-PT84 group, S-PT84 was used in a cell count that corresponded to the number of cells contained in the fermented sesame/soybean/Kombu. The control group was allowed to ingest ad libitum the basic feed AIN-93N, and the other groups were allowed to ingest ad libitum a special feed prepared by mixing the basic feed AIN-93N with the ingredients specified above. One week after the start of ingestion, the small intestine was extracted and its interior was washed with PBS; thereafter, four volumes of PBS containing 0.05% Tween 20 was added and the mixture was homogenized with a homogenizer POLYTRON. Following centrifugation, the supernatant was recovered to obtain an extract of the small intestine's wall. The IgA level in the extract of the small intestine's wall was measured by ELISA (Purified IgA Antibody was used as a primary antibody whereas Biotin Conjugate IgA Antibody was used as a secondary antibody (both antibodies were products of Southern Biotech); for color development, Avidin-Horseradish peroxide Conjugate was used).

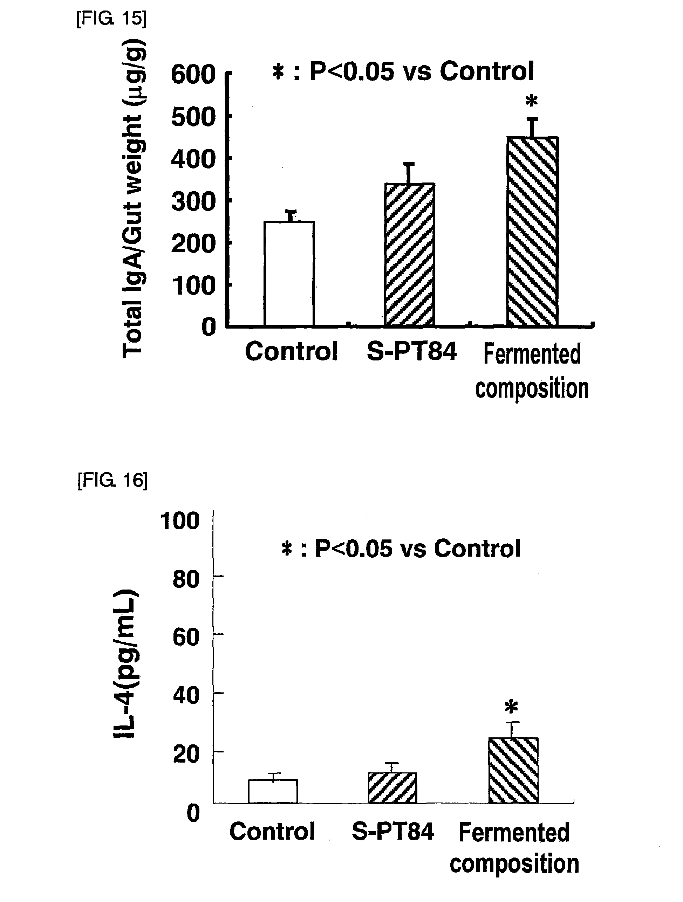

The IgA level is shown in FIG. 15. As is clear from FIG. 15, the IgA level in the extract of the small intestine's wall increased significantly in the fermented sesame/soybean/Kombu ingesting group as compared with the control group. This action of the fermented sesame/soybean/Kombu of the present invention was stronger than in the case where S-PT84 was ingested alone.

Example 10 Action of Promoting Cytokine Production from Intestinal Immunocompetent cells by Oral Intake of Fermented Sesame/Soybean/Kombu

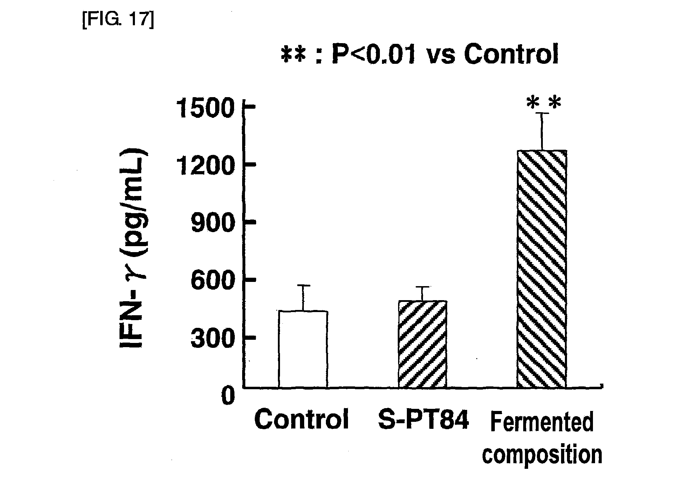

C57BL/6 mice (7-week old, male) were divided into three groups such that each group had almost the same average weight. These groups were as follows: a control group (7 animals), an S-PT84 (dry dead cells mixed in 0.0075%) ingesting group (9 animals), and a group ingesting the fermented sesame/soybean/Kombu obtained in Example 2 (mixed in 5%) (9 animals: Fermented Composition). The control group was allowed to ingest ad libitum the basic feed AIN-93N, and the other groups were allowed to ingest ad libitum a special feed prepared by mixing the basic feed AIN-93N with the ingredients specified above. One week later, Peyer's patch cells were extracted and lightly compressed on a cell strainer (70 μm, product of BD Falcon) in a cell culturing medium (RPMI 1640 medium containing 10% fetal bovine serum and a 0.2% antibiotic-antifungal agent mixed solution (product of Nakalai Tesque)), to recover the cells and samples of Peyer's patch cells were prepared. In a 10% FBS supplemented RPMI medium, 2.5 μg/mL of concanavalin A was allowed to act on $5 \times 10^6$ cells for 24 hours, and the amounts of IL-4 and INF-γ produced in the supernatant were measured by ELISA with OptEIA (BD Pharmingen). The test results for the amount of IL-4 production are shown in FIG. 16 and the test results for the amount of INF-γ production are shown in FIG. 17.

As is clear from FIGS. 16 and 17, in the Peyer's patch cells prepared from the mice that ingested the fermented sesame/soybean/Kombu, the amounts of INF-γ and IL-4 production rose significantly. On the other hand, no such action was recognized in the lactic acid bacteria ingesting group (S-PT84).

From the results of Examples 9 and 10, it became clear that the intestinal tract immunostimulating action was found only when the fermented sesame/soybean/Kombu was ingested orally, but not found when the lactic acid bacteria alone was ingested. This effect of the fermented composition of the present invention can hardly be anticipated from the action of the starting materials for fermentation.

Example 11 Anti-tumor Effect of the Kombu Ferment

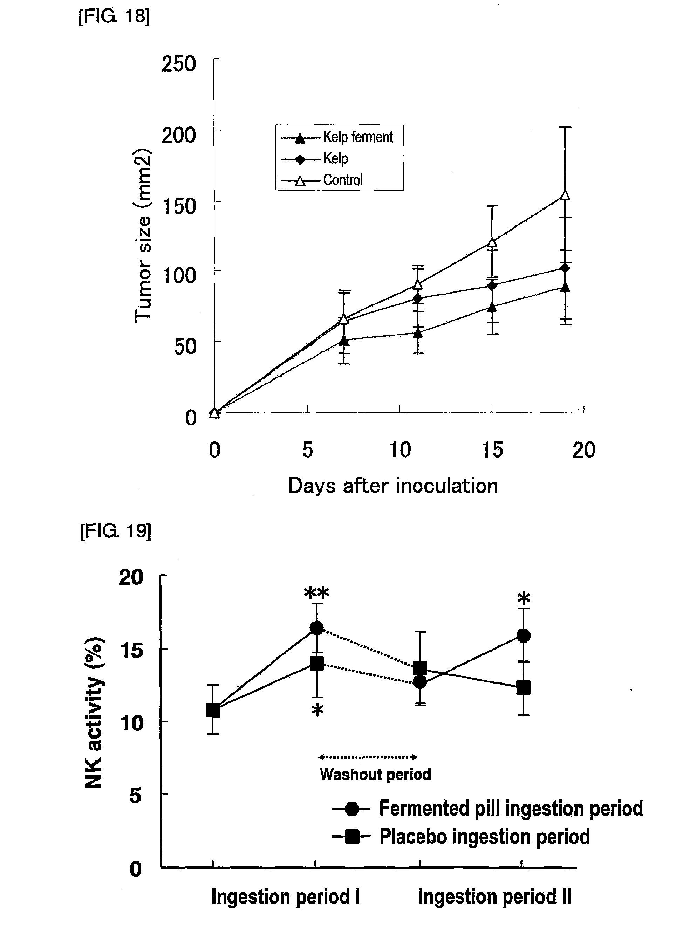

ddy Mice (Japan SLC, Inc.) were divided into four groups (10 animals per group) and forcibly administered orally daily with water (0.6 mL) or 500 mg/kg of the unfermented Kombu (500 mg/kg) or the fermented Kombu (500 mg/kg). At day 2 after the start of forcible oral administration, the chest of each mouse was inoculated subcutaneously

with 1 x 10[6] Sarcoma 180 cells to prepare Sarcoma 180 bearing mice. The Sarcoma 180 bearing mice were continuously subjected to oral administration for 18 days. During the test period, the tumor diameters were measured and the tumor size was determined in accordance with formula (I) set forth below (FIG. 18). As a result, the tumor size in each of the unfermented Kombu administered group and the fermented Kombu administered group varied at lower levels than the control group administered with only water; in the fermented Kombu administered group, the tumor size was significantly small at days 11, 15 and 19 after tumor inoculation. It was thus found that the fermented Kombu had a higher antitumor effect.

$$(I) \text{ Tumor size } (mm^2) = \text{Max. dia. (mm) x min. dia. x } 3.14$$

Example 12

Using the composition prepared in Example 2 by fermenting sesame, soybeans and Kombu with lactic acid bacteria (fermented sesame/soybean/Kombu), pills containing the fermented sesame/soybean/Kombu (which are hereinafter referred to as fermented pills) were produced in accordance with the recipe shown in Table 4 (weight of one pills: 350 mg). Specifically, the fermented sesame/soybean/Kombu was mixed with additional starting materials such as excipients and sweeteners for taste adjustment, blended with a blender, and molded into pills through a pill-making machine. Thereafter, the pills were dried to a suitable extent, and their surfaces were coated with shellac. Note that, considering the nature of the fermented sesame/soybean/Kombu, the step of buffing the surface of uncoated pills, commonly performed in the manufacture of pills, was omitted. In addition, pills not containing the fermented sesame/soybean/Kombu (but in the same form as the fermented pills) were produced as placebo. The recipes for the fermented pill and the placebo are shown in Table 4.

[Table 4]

| Compositional Table (w//w%) | | |
|---|---|---|
| | Fermented pills | Placebo |
| Fermented sesame/soybean/Kombu | 45.0 | 0 |
| Excipients (starch, etc.) | 18.5 | 63.5 |
| Sweeteners (honey, anhydrous crystalline maltose, xylooligosaccharide, etc.) | 36.5 | 36.5 |
| Total | 100 | 100 |

The two kinds of pills were administered to 40 in-house healthy volunteers. They ingested 30 pills a day (4.7 g/day as the fermented sesame/soybean/Kombu), and a study was made to evaluate their immunomodulatory actions. The ingestion test was conducted by a crossover design. To be more specific, the subjects were divided into two groups such that they would not have any statistical difference in terms of NK activity, age and sex; the subjects were initially allowed to ingest either the fermented pills or the placebo in the same form for four weeks (ingestion period I), and a 6-week washout period was provided. Thereafter, the samples to be ingested were switched and the subjects were allowed to ingest either the placebo or the fermented pills for a month (ingestion period II). Before and after each of the ingestion periods, blood was sampled and leukocytes were prepared by Ficoll-Hypaque density-gradient centrifugal sedimentation (Nycomed Pharma), and their NK activity was measured. As for the NK activity, cytotoxicity activity with target cells K562 was measured by the PINK method at a leukocyte to K562 ratio of 20:1. In addition, the leukocytes were cultured in the presence of PHA (phytohemagglutinin) for 22 hours, and the amounts of IFN-γ and IL-4 production in the culture supernatant were measured for analysis of Th1/Th2 balance.

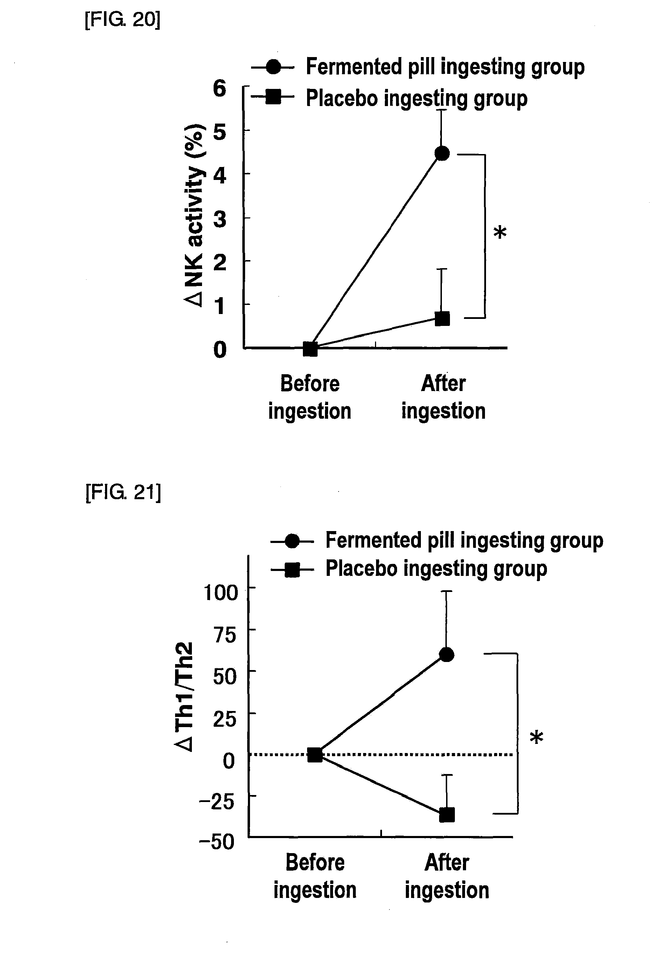

The test results for NK activity are shown in FIG. 19. In both ingestion periods I and II, the NK activity rose significantly by ingesting the fermented pills. Further, the amounts of change in NK activity during ingestion periods I and II (the difference between the initial value and the value after ingestion: ΔNK activity) were determined and the average was computed. The results are shown in FIG. 20. The fermented pills ingesting group showed a significantly great amount of change in NK activity as compared with the placebo ingesting group.

In the next step, the amounts of IFN-γ and IL-4 production from the leukocytes after PHA stimulation were measured, the changes in Th1/Th2 ratio (the amount of IFN-γ production/the amount of IL-4 production) during ingestion periods I and II (the difference between the initial value and the value after ingestion: ΔTh1/Th2) were determined, and the average was computed. The results are shown in FIG. 21. The fermented pills ingesting group showed a significantly great amount of change in Th1/Th2 as compared with the placebo group, suggesting that the ingestion of the fermented pills would bring about a Th1 dominant state.

The present invention is by no means limited to the embodiments described above and it should be understood that various modifications are possible within the scope of claims, and even embodiments obtained by appropriate combinations of the technical means disclosed in the different embodiments are also included within the technical scope of the present invention.

## Claims

1. A composition having an immunomodulatory action that comprises Kombu fermented with lactic acid bacteria.

2. A composition according to claim 1, wherein the immunomodulatory action is an immunostimulating action and/or an anti-allergic action.

3. A composition according to claim 1 or 2, wherein the lactic acid bacteria are those of plant origin.

4. A composition comprising Kombu fermented with lactic acid bacteria, sesame fermented with lactic acid bacteria, and soybean(s) fermented with lactic acid bacteria.

5. A composition according to claim 4, wherein the lactic acid bacteria are those of plant origin.

6. A composition according to claim 4 or 5, which has an immunomodulatory action.

7. A composition according to claim 6, wherein the immunomodulatory action is an immunostimulating action and/or an anti-allergic action.

8. A composition according to any one of claims 1 to 7, which is a food or beverage, a food additive, a health food, or a pharmaceutical composition.

9. A composition according to any one of claims 1 to 8, which is in the form of a pill.

[FIG. 1]

[FIG. 2]

[FIG. 3]

[FIG. 4]

*: P<0.05 vs Cont

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

[FIG. 16]

[FIG. 17]

** : P<0.01 vs Control

[FIG. 18]

[FIG. 19]

[FIG. 20]

[FIG. 21]

<div align="center">

**INTERNATIONAL SEARCH REPORT**

</div>

| | International application No. |
|---|---|
| | PCT/JP2006/304087 |

A.  CLASSIFICATION OF SUBJECT MATTER
*A61K36/03*(2006.01), *A61K36/06*(2006.01), *A61K35/74*(2006.01), *A61K36/48*
(2006.01), *A61P11/06*(2006.01), *A61P17/00*(2006.01), *A61P31/00*(2006.01),
*A61P31/18*(2006.01), *A61P35/00* (2006.01), *A61P37/02*(2006.01),
According to International Patent Classification (IPC) or to both national classification and IPC

B.  FIELDS SEARCHED

Minimum documentation searched (classification system followed by classification symbols)
A23L1/30, A23L2/52, A61K35/74, A61K36/48, A61K36/03, A61K36/06, A61P11/06,
A61P17/00, A61P31/00, A61P31/18, A61P35/00, A61P37/02, A61P37/04, A61P37/08

Documentation searched other than minimum documentation to the extent that such documents are included in the fields searched
Jitsuyo Shinan Koho           1922-1996   Jitsuyo Shinan Toroku Koho   1996-2006
Kokai Jitsuyo Shinan Koho    1971-2006   Toroku Jitsuyo Shinan Koho   1994-2006

Electronic data base consulted during the international search (name of data base and, where practicable, search terms used)
BIOSIS/MEDLINE/WPIDS(STN), CAplus(STN), JMEDPlus(JOIS), JSTPlus(JOIS)

C.  DOCUMENTS CONSIDERED TO BE RELEVANT

| Category* | Citation of document, with indication, where appropriate, of the relevant passages | Relevant to claim No. |
|---|---|---|
| P,X | Takayuki IZUMO et al., "Gozukon Hakkobutsu no Men'eki Chosetsu Kino I Stress ni yoru Men'eki Teika ni Taisuru Koka", Nippon Nogei Kagakukai Taikai Koen Yoshishu, 05 March, 2005 (05.03.05), Vol.2005, page 114 | 1-9 |
| P,X | Yuji NONAKA et al., "Gozukon Hakkobutsu no Men'eki Chosetsu Kino II Hito no Men'eki Kino ni Ataeru Eikyo", Nippon Nogei Kagakukai Taikai Koen Yoshishu, 05 March, 2005 (05.03.05), Vol.2005, page 114 | 1-9 |
| P,X | JP 2006-50915 A  (Nippon Suisan Kaisha, Ltd.), 23 February, 2006 (23.02.06), (Family: none) | 1-3,8,9 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 March, 2006 (29.03.06) | 16 May, 2006 (16.05.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
|---|
| PCT/JP2006/304087 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 2004-313032 A   (Sadaharu YOKOYAMA),<br>11 November, 2004 (11.11.04),<br>Claims 2, 3<br>(Family: none) | 1-3,8,9<br>4-7 |
| X<br>Y | Takuo WADA, "GABA Gan'yu Nyusankin Hakko Konbu<br>Chomiryo no Teimi Koka", Food Research, 01<br>February, 2005 (01.02.05), No.596, pages 12<br>to 16 | 1-3,8,9<br>4-7 |
| X<br>Y | JP 2003-201 A   (Incorporated Administrative<br>Agency Fisheries Research Agency),<br>07 January, 2003 (07.01.03),<br>(Family: none) | 1-2,8,9<br>3-7 |
| Y | CN 1389228 A   (Peop.Rep. China),<br>08 January, 2003 (08.01.03),<br>:Chem, abs. AN.140:241004<br>(Family: none) | 1-9 |
| Y | Yuji NONAKA et al., "Shokubutsusei Nyusankin<br>no Men'eki Chosetsu Kino no Kento (1)", The<br>Japanese Society of Nutrition and Food Science<br>Sokai Koen Yoshishu, 2004, Vol.58th, page 124,<br>2F-5a | 1-9 |
| Y | Takayuki IZUMO et al., "Shokubutsusei Nyusankin<br>no Men'eki Chosetsu Kino no Kento (2)", The<br>Japanese Society of Nutrition and Food Science<br>Sokai Koen Yoshishu, 2004, Vol.58th, page 124,<br>2F-6a | 1-9 |
| Y | WO 01/13925 A1   (Takara Bio Inc.),<br>01 March, 2001 (01.03.01),<br>& EP 1226826 A1          & AU 6593400 A<br>& CN 1382055 A | 1-9 |
| Y | JP 2004-173692 A   (Japan Applied Microbiology<br>Research Institut),<br>24 June, 2004 (24.06.04),<br>(Family: none) | 4-9 |
| Y | J.G. Leblanc et al, A Novel Functional Soy-<br>based Food Fermented by Lactic Acid Bacteria:<br>Effect of Heat Treatment, J. Food Science, 2004,<br>Vol.69, Nr.8, pages 246 to 250 | 4-9 |
| Y | Toshimi ISHII et al., "Daizu Hakkobutsu<br>Seibun ni yoru Rat Fukukonai Saibo o<br>Mochiita Histamine Yuri Yokusei Koka",<br>Nippon Yakugakukai Nenkai Koen Yoshishu,<br>2001, Vol.121st, No.4, page 174 | 4-9 |

Form PCT/ISA/210 (continuation of second sheet) (April 2005)

**INTERNATIONAL SEARCH REPORT**

International application No.

PCT/JP2006/304087

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-26582 A (Microbio Co., Ltd.),<br>29 January, 2003 (29.01.03),<br>Par. No. [0025]<br>& EP 1243274 A2　　　& EP 1243274 A3<br>& US 2002/182274 A1　& US 2003/3169 A1<br>& US 2003/8023 A1　　& US 6685973 B1<br>& CA 2342708 A　　　& CN 1375314 A | 4-9 |
| Y | JP 9-238647 A (Yakult Honsha Co., Ltd.),<br>16 September, 1997 (16.09.97),<br>(Family: none) | 4-9 |
| Y | Yasuko FUKUDA et al., "Goma to Daizu no<br>Biseibutsu Shori ni yoru Atarashii Kinosei no<br>Hatsugen to Komugiko Seihin eno Riyo", Annual<br>Report/ the Iijima Memorial Foundation for<br>Promotion of Food Science and Technology, 1997,<br>Vol.1995, pages 244 to 249, Fig. 1 | 4-9 |
| Y | SOU Koan et al., "Rorei Mouse ni Okeru Hakko<br>Daizu Nyusankin Baiyobutsu no Chokan Men'eki<br>Fukatsu Sayo", The Japanese Society of<br>Nutrition and Food Science Sokai Koen<br>Yoshishu 2003, Vol.57th, page 70 | 4-9 |
| Y | JP 10-72362 A (Kyodo Nyugyo Kabushiki Kaisha),<br>19 March, 1998 (19.03.98),<br>(Family: none) | 1-9 |
| Y | JP 6-292529 A (Kabushiki Kaisha Horiuchi),<br>21 October, 1994 (21.10.94),<br>& JP 2846547 B2 | 1-9 |

Form PCT/ISA/210 (continuation of second sheet) (April 2005)

**INTERNATIONAL SEARCH REPORT**

International application No.

PCT/JP2006/304087

Continuation of A. CLASSIFICATION OF SUBJECT MATTER
(International Patent Classification (IPC))

*A61P37/04*(2006.01), *A61P37/08*(2006.01), *A23L1/30*(2006.01), *A23L2/52*
(2006.01)

(According to International Patent Classification (IPC) or to both national classification and IPC)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003000201 A
- JP 2846547 B
- JP 2005333919 A
- JP 2004173692 A
- JP 2003335695 A